# EUROPÄISCHE PATENTANMELDUNG

(11) **EP 0 557 821 A1**
(43) Veröffentlichungstag der Anmeldung: **01.09.1993**
(21) Anmeldenummer: 93102223.0
(22) Anmeldetag: 12.02.1993
(51) Int. Cl.: C07C 49/403, C07C 45/78

(54) **Verfahren zur Reinigung von Cyclohexanon**

(30) Priorität: 25.02.1992 DE 4205633
(71) Anmelder: BAYER ANTWERPEN N.V., 2040 Antwerpen (BE)
(72) Erfinder: Meier, Heinz-Peter, W-2950 Kapellen (BE); van Esbroeck, Jan, Dr., B-2920 Kalmthout (BE); Terweduwe, Eddy, B-2600 Berchem (BE)
(74) Vertreter: Zobel, Manfred, Dr.

(57) **Zusammenfassung**

Cyclohexanon, das durch Dehydrierung eines Cyclohexanon/Cyclohexanol-Gemisch erhalten wurde, wird von Nebenprodukten dadurch weitgehend befreit, daß man ein solches Dehydrierungsgemisch in der Gas- oder Flüssigphase mit Wasserstoff an einem Hydrierkatalysator bei 20 bis 180°C und einem Druck von 0,1 bis 15 bar behandelt.

## Beschreibung

Die Erfindung betrifft ein Verfahren zur Reinigung von Cyclohexanon, das durch Dehydrierung eines Cyclohexanon/Cyclohexanol-Gemisches erhalten wurde, mit Wasserstoff bei erhöhter Temperatur und vermindertem bis erhöhtem Druck.

Ein im erfindungsgemäßen Verfahren einsetzbares Cyclohexanon kann beispielsweise so erhalten werden, daß man Cyclohexan zunächst mit Sauerstoff in Gegenwart von katalytisch wirkenden Metallsalzen oder in Gegenwart von Borsäure oxidiert und hierbei ein Reaktionsgemisch erhält, welches unter anderem Cyclohexanon, Cyclohexanol, gegebenenfalls dessen Borsäureester und Cyclohexylhydroperoxid enthält. Nach der thermischen oder katalytisch-hydrierenden Zersetzung der Peroxide und nach der Abtrennung der genannten Bestandteile vom Oxidationsgemisch erhält man ein Cyclohexanon/Cyclohexanol-Gemisch. Für den Fall, daß Cyclohexanon das gewünschte Endprodukt ist, wird dieses Gemisch, gegebenenfalls nach vorheriger Abtrennung von bereits vorliegendem Cyclohexanon, dehydriert, so daß der Cyclohexanolanteil ebenfalls in Cyclohexanon übergeht. Ein solches Cyclohexanon enthält jedoch noch Verunreinigungen im Bereich von 0,5 bis 5 ^{o}/oo, die zum Teil ungesättigter Natur sind, zum Teil aber auch nicht vollständig bekannt sind.

Des weiteren kann durch Hydrierung von Phenol ein Cyclohexanon/Cyclohexanol-Gemisch erhalten werden, dessen Cyclohexanolanteil ebenfalls in der beschriebenen Weise zu Cyclohexanon dehydriert werden kann. Auch ein solches Cyclohexanon enthält unerwünschte, zum Teil ungesättigte Begleitstoffe im genannten Bereich.

Die in einem solchen Cyclohexanon enthaltenen Verunreinigungen können selbst unter großem Aufwand nicht oder nur teilweise entfernt werden. Sie stören jedoch die folgende Weiterverarbeitung des Cyclohexanons, beispielsweise bei der anschließenden Herstellung von Caprolactam.

Zur Abtrennung der genannten Verunreinigungen, gegebenenfalls nach vorangegangener Isomerisierung, wurden bereits erhebliche Anstrengungen unternommen. So wurde beschrieben, das in obiger Weise erhaltene Cyclohexanon mit Alkalihydroxiden, Alkalialkoholaten, Phenolaten oder deren Lösungen oder mit basischen Ionenaustauschern zu behandeln und anschließend zu rektifizieren, bzw. Hilfsstoffe der genannten Art bei der Rektifikation zuzusetzen (DE-AS 1 188 584). Die Anwesenheit von alkalisch reagierenden Verbindungen bewirkt jedoch unkontrollierbare Kondensationsreaktionen, die nicht nur zu weiteren Verunreinigungen führen sondern auch erhebliche Ausbeuteverluste darstellen.

Da ein Teil der störenden Verbindungen ungesättigter Natur ist, ist bereits versucht worden, diese unter hydrierenden Bedingungen entweder in Cyclohexanon oder in leichter abtrennbare Verbindungen umzuwandeln. Dies betrifft beispielsweise die besonders störenden Verbindungen Cyclohexen und die verschiedenen Cyclohexenone. In allen bisher bekannten Hydrierverfahren ist jedoch stets ein großer Anteil des Cyclohexanons wieder zu Cyclohexanol reduziert worden, z.B. gemäß BE 714 128, worin eine katalytische Gasphasenhydrierung vor der destillativen Reinigung beschrieben wird; eine Überprüfung zeigte, daß hierbei weitere Nebenprodukte entstehen, die zusätzlich entfernt werden müssen. Es schien daher, daß eine hydrierende Reinigung auf verschiedenen Stufen des Verfahrens zur Cyclohexanon-Herstellung erfolglos bleiben würden. Gemäß EP 411 455 wurde daher die störende Auswirkung der Verunreinigungen auf die Qualität von Caprolactam erst an diesem Produkt, also durch katalytische Hydrierung einer wäßrigen Caprolactamlösung, zu lösen versucht.

Überraschend wurde nun gefunden, daß die störenden Verunreinigungen im Cyclohexanon unter milden Bedingungen sowohl in der Gasphase als auch in der Flüssigphase unter den erfindungsgemäßen Bedingungen entfernt werden können, wenn ein oben beschriebenes Dehydrierungsgemisch als Substrat eingesetzt wird.

Die Erfindung betrifft demnach ein Verfahren zur Reinigung von Cyclohexanon, das durch Dehydrierung eines Cyclohexanon/Cyclohexanol-Gemisches erhalten wurde, das dadurch gekennzeichnet ist, daß das Dehydrierungsgemisch in der Gas- oder Flüssigphase mit Wasserstoff an einem Hydrierkatalysator bei 20 bis 180°C, bevorzugt 50 bis 150°C, und bei einem H₂-Druck von 0,1 bis 15 bar, bevorzugt 1,1 bis 5 bar behandelt wird.

Als Wasserstoff kann einer aus beliebiger Quelle genommen werden. In bevorzugter Weise wird jedoch der beim vorangegangenen Dehydrierschritt erhaltene Wasserstoff eingesetzt.

In bevorzugter Weise wird das erfindungsgemäße Verfahren in der Flüssigphase durchgeführt. Hierbei wird das Dehydriergemisch nicht entgast, sondern der aus der Dehydrierung stammende und noch im flüssigen Dehydriergemisch enthaltene Wasserstoff wird verwendet. Sollte dieser bereits vorhandene Wasserstoff nicht ausreichen, was an einer unzureichenden Beseitigung der störenden Nebenprodukte erkennbar ist, wird weiterer Wasserstoff aus einer externen Quelle zugesetzt. Ein solcher externer Wasserstoff kann mit dem erfindungsgemäß zu behandelnden Dehydriergemisch im Gleich- oder im Gegenstrom geführt werden.

Als Hydrierkatalysatoren werden solche eingesetzt, die ein Element der VIII. Nebengruppe des Periodensystems der Elemente (Mendelejew) enthalten, also Fe, Co, Ni, Ru, Rh, Pd, Os, Ir oder Pt. In bevorzugter Weise werden die Edelmetalle der Platingruppe, in besonders bevorzugter Weise Palladium eingesetzt. Solche Katalysatoren werden in einer dem Fachmann geläufigen Weise auf Trägern, wie SiO₂, Al₂O₃, Bims und anderen angeordnet. Der Edelmetallgehalt beträgt 0,01 bis 10 %, bevorzugt 0,1 bis 3 % des Gesamtgewichtes des Katalysators. Es ist dem Fachmann weiterhin bekannt, daß solche Hydrierkatalysatoren ferner Dotierungs- und Promotorelemente enthalten können, beispielsweise Pb, V und andere.

Katalysatoren dieser Art werden beispielsweise bei der Herstellung von Anilin durch Gasphasenhydrierung von Nitrobenzol eingesetzt, wobei der aromatische Kern nicht angegriffen wird, aber die funktionelle NO₂-Gruppe zur NH₂-Gruppe reduziert wird. Es ist daher erstaunlich, daß bei dem erfindungsgemäß zu reinigenden Cyclohexanon die funktionelle Ketogruppe erhalten bleibt.

Zur Durchführung des erfindungsgemäßen Verfahrens kann ein einfacher rohrförmiger Reaktor verwendet werden, in welchem der Katalysator in fester Form oder als lose Schüttung angeordnet ist. Das zu behandelnde Dehydriergemisch wird entweder von unten her durch die Katalysatorfüllung gepumpt oder aber von oben über diese Füllung geleitet.

Das erfindungsgemäße Verfahren wird bei 20 bis 180°C, bevorzugt bei 50 bis 150°C und einem Druck von 0,1 bis 15 bar, bevorzugt 1,1 bis 5 bar durchgeführt. Es wird eine Verweilzeit des Dehydriergemisches am Katalysator von 2 bis 100 Minuten, bevorzugt 3 bis 30 Minuten eingestellt. Der dem Dehydriergemisch zugesetzte Wasserstoff beträgt 0 bis 100 Volumenteile H₂ unter Normalbedingungen je Volumen Flüssigdurchsatz, wobei auch bei der Durchführung in der Gasphase das nach der Dehydrierung noch gasförmig vorliegende bzw. zunächst flüssig vorliegende und wieder zu verdampfende Dehydriergemisch mit seinem Flüssigvolumen verstanden wird. Die Untergrenze von 0 Volumenteile Wasserstoffe zeigt an, daß, wie oben bereits beschrieben, der im flüssigen Dehydriergemisch gelöste Wasserstoff ausreichen kann. In bevorzugter Weise werden 0,5 bis 5 Volumenteile Wasserstoff unter Normalbedingungen je Volumenteil Flüssig

durchsatz zugesetzt. Eine bevorzugte Durchführungsform ist die Gegenstromfahrweise, bei der Wasserstoff von unten durch die Katalysatorschüttung geleitet wird und das zu reinigende Dehydriergemisch in flüssiger Phase von oben über den Katalysator rieselt.

Es ist erstaunlich und war nicht vorherzusehen, daß bei einer Hydrierung unter den obengenannten Bedingungen kein Verlust an Cyclohexanon und keine Rückhydrierung zum Cyclohexanol eintritt. Der Umsatz an ungesättigten Verbindungen im erfindungsgemäßen Verfahren kann bis zu 99 % ihrer ursprünglichen Menge betragen.

### Beispiele

Alle Versuche wurden in einem Metallrohr von 2,85 m Länge und 25 mm Durchmesser durchgeführt. Der jeweilige Katalysator wurde zwischen Siebplatten als lose Schüttung eingebracht. Der Bodenteil der Kolonne wurde so konstruiert, daß dort einerseits Wasserstoff eingebracht werden konnte und gleichzeitig ein dehydriertes Rohgemisch bei Gleichstromfahrweise eingepumpt (Überlauf am oberen Kolonnenteil) oder aber bei Gegenstromfahrweise ein nachhydriertes Rohgemisch über eine Standregelung abgelassen werden konnte. Bei Gasphasenbetrieb konnte das Wasserstoff/Cyclohexanon/Cyclohexanol-Gemisch sowohl von unten als auch von oben in die Kolonne eingebracht werdend Am Kopfteil der Kolonne befand sich ein Ein-bzw. Überlauf mit einem Kühler/Kondensator. Die entsprechenden Gas- und Flüssigkeitsströme wurden über Rotameter gemessen. Der Umsatz wurde aus den gaschromatographisch gemessenen Konzentrationsänderungen bei den jeweiligen Substanzen ermittelt.

### Beispiel 1

| | |
|---|---|
| Anolon gasf. Menge: | 3,8 l/h |
| Gehalt ungesättigte Verbindungen: | 1201 ppm |
| Wasserstoff Menge: | 2,7 l/h |
| Art: | aus Dehydrierung |
| Katalysator: | 2,2 % Pd |
| Verweilzeit: | 13 Minuten |
| Umsatz ungesättigte Verbindungen: | 48 % |
| Temperatur: | 124° |
| Druck: | 1,5 bar |

### Beispiel 2

| | |
|---|---|
| Anolon flüssig Menge: | 1 l/h |
| Gehalt ungesättigte Verbindungen: | 1331 ppm |
| Wasserstoff Menge: | 5 l/h |
| Art: | rein |
| Katalysator: | 2,2 % Pd |
| Verweilzeit: | 8 Minuten |
| Umsatz ungesättigte Verbindungen: | 45 % |
| Temperatur: | 25° |
| Druck: | 1,1 bar |

### Beispiel 3

| | |
|---|---|
| Anolon flüssig Menge: | 7 l/h |
| Gehalt ungesättigte Verbindungen: | 1080 ppm |
| Wasserstoff Menge: | 6 l/h |
| Art: | rein |
| Katalysator: | 2,2 % Pd |
| Verweilzeit: | 14 Minuten |
| Umsatz ungesättigte Verbindungen: | 92 % |
| Temperatur: | 68° |
| Druck: | 1,1 bar |

### Beispiel 4

| | |
|---|---|
| Anolon flüssig Menge: | 10 l/h |
| Gehalt ungesättigte Verbindungen: | 1135 ppm |
| Wasserstoff Menge: | 10 l/h |
| Art: | aus Dehydrierung |
| Katalysator: | 1,2 % Pd + V + Pb |
| Verweilzeit: | 10 Minuten |
| Umsatz ungesättigte Verbindungen: | 73 % |
| Temperatur: | 105° |
| Druck: | 1,3 bar |

### Beispiel 5

| | |
|---|---|
| Anolon flüssig Menge: | 10 l/h |
| Gehalt ungesättigte Verbindungen: | 1132 ppm |
| Wasserstoff Menge: | 10 l/h |
| Art: | rein |
| Katalysator: | 1,2 % Pd + V + Pb |
| Verweilzeit: | 10 Minuten |
| Umsatz ungesättigte Verbindungen: | 83 % |
| Temperatur: | 105° |
| Druck: | 1,3 bar |

### Beispiel 6

| | |
|---|---|
| Anolon flüssig Menge: | 6 l/h |
| Gehalt ungesättigte Verbindungen: | 883 ppm |
| Wasserstoff Menge: | 0 l/h |
| Katalysator: | 2,2 % Pd |
| Verweilzeit: | 17 Minuten |
| Umsatz ungesättigte Verbindungen: | 22 % |
| Temperatur: | 95° |
| Druck: | 1,1 bar |

### Beispiel 7

| | |
|---|---|
| Anolon flüssig Menge: | 15 l/h |
| Gehalt ungesättigte Verbindungen: | 962 ppm |
| Wasserstoff Menge: | 20 l/h |
| Art: | aus Dehydrierung |
| Katalysator: | 0,65 % Pd |
| Verweilzeit: | 7 Minuten |
| Umsatz ungesättigte Verbindungen: | 99 % |
| Temperatur: | 110° |
| Druck: | 1,3 bar |

### Beispiel 8

| | |
|---|---|
| Anolon flüssig Menge: | 15 l/h |
| Gehalt ungesättigte Verbindungen: | 1020 ppm |
| Wasserstoff Menge: | 20 l/h |
| Art: | aus Dehydrierung |
| Katalysator: | 0,3 % Pd |
| Verweilzeit: | 7 Minuten |
| Umsatz ungesättigte Verbindungen: | 97 % |
| Temperatur: | 110° |
| Druck: | 1,3 bar |

## Patentansprüche

1. Verfahren zur Reinigung von Cyclohexanon, das durch Dehydrierung eines Cyclohexanon/Cyclohexanol-Gemisches erhalten wurde, dadurch gekennzeichnet, daß das Dehydrierungsgemisch in der Gas- oder Flüssigphase mit H₂ an einem Hydrierkatalysator bei 20 bis 180°C, bevorzugt 50 bis 150°C, und einem H₂-Druck von 0,1 bis 15 bar, bevorzugt 1,1 bis 5 bar behandelt wird.

2. Verfahren nach Anspruch 1, dadurch gekennzeichnet, daß in der Flüssigphase gearbeitet wird.

3. Verfahren nach Anspruch 1, dadurch gekennzeichnet, daß als Hydrierkatalysator ein Edelmetall-Träger-Katalysator mit einem Edelmetall aus der Platingruppe, bevorzugt mit Palladium eingesetzt wird.

4. Verfahren nach Anspruch 3, dadurch gekennzeichnet, daß der Gehalt an Edelmetall 0,01 bis 10 Gew.-%, bevorzugt 0,1 bis 3 Gew.-%, bezogen auf das Gesamtgewicht des Katalysators, beträgt.

5. Verfahren nach Anspruch 1, dadurch gekennzeichnet, daß die Behandlung während einer Zeit von 2 bis 100 Minuten, bevorzugt 3 bis 30 Minuten durchgeführt wird.

6. Verfahren nach Anspruch 2, dadurch gekennzeichnet, daß beim Arbeiten in der Flüssigphase der Wasserstoff von unten durch den Katalysator geführt wird und das flüssige Dehydriergemisch von oben über den Katalysator geleitet wird.
